(19) Europäisches Patentamt
European Patent Office
Office européen des brevets

(11) EP 1 236 460 B1

(12) **EUROPÄISCHE PATENTSCHRIFT**

(45) Veröffentlichungstag und Bekanntmachung des Hinweises auf die Patenterteilung:
**26.11.2003 Patentblatt 2003/48**

(51) Int Cl.[7]: **A61K 7/13**, C07D 231/38

(21) Anmeldenummer: **01124089.2**

(22) Anmeldetag: **10.10.2001**

(54) **Verbrückte Diaminopyrazole und diese Verbindungen enthaltende Färbemittel**

Bridged diamino pyrazoles and colouring agents containing them

Diamino pyrazoles pontes et colorants les contenant

(84) Benannte Vertragsstaaten:
**AT BE CH CY DE DK ES FI FR GB GR IE IT LI LU MC NL PT SE TR**

(30) Priorität: **01.03.2001 DE 10109807**

(43) Veröffentlichungstag der Anmeldung:
**04.09.2002 Patentblatt 2002/36**

(73) Patentinhaber: **Wella Aktiengesellschaft
64274 Darmstadt (DE)**

(72) Erfinder:
- **Göttel, Otto, Dr.
1723 Marly (CH)**
- **Pirrello, Aline
1762 Givisiez (CH)**
- **Hayoz, André
1724 Senèdes (CH)**
- **Morand, Emmanuel
1740 Neyruz (CH)**

(56) Entgegenhaltungen:
**EP-A- 0 740 931**
**WO-A-97/42173**
**WO-A-99/11231**
**DE-B- 1 295 560**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 236 460 B1

## Beschreibung

Gegenstand der vorliegenden Anmeldung sind neue 4,5-Diamino-pyrazole sowie deren physiologisch verträgliche Salze mit organischen oder anorganischen Säuren. Ein weiterer Gegenstand sind diese Verbindungen als Farbstoff-Vorstufen enthaltende Färbemittel.

Auf dem Gebiet der traditionellen Haarfärbung haben Oxidationsfarbstoffe eine wesentliche kosmetische Bedeutung erlangt. Die Färbung entsteht hierbei durch Reaktion bestimmter Entwickler- und Kupplersubstanzen in Gegenwart eines Oxidationsmittels. Von besonderer Bedeutung sind nach wie vor Haarfärbemittel zur Färbung im Naturtonbereich. Daneben lassen sich durch Kombination geeigneter Oxidationsfarbstoff-Vorstufen auch zeitgemäß modische Farbnuancen erzeugen. Gegenwärtig im Modetrend liegen abgewandelte Naturtöne wie beispielsweise Brauntöne mit ausgeprägten Aubergine- oder Kupfer-Nuancen, insbesondere aber leuchtende Rottöne.

Neben der Erzeugung von Farbeffekten werden an Oxidationsfarbstoffe, die zur Behandlung menschlicher Haare vorgesehen sind, sehr hohe Anforderungen gestellt. Die Farbstoffe müssen einerseits in toxikologischer und dermatologischer Hinsicht unbedenklich sowie nicht sensibilisierend sein. Außerdem ist es erforderlich, dass durch Kombination geeigneter Entwickler- und Kupplerkomponenten eine breite Palette verschiedener Farbnuancen erzeugt werden kann. Ferner wird für die erzielten Haarfärbungen eine gute Wasch-, Licht-, Schweiß-, Dauerwell-, Säure-, Basen- und Reibeechtheit gefordert. In jedem Fall müssen solche Haarfärbungen unter den heute üblichen Alltagsbedingungen mindestens vier bis sechs Wochen stabil bleiben.

Zur Abdeckung des zunehmend wichtigen Rotbereichs wurde in der Vergangenheit überwiegend 4-Aminophenol als Entwickler verwendet. Wegen Bedenken in bezug auf die physiologische Verträglichkeit dieser Substanz wurden auch Pyridin- und Pyrimidinderivate eingesetzt, die allerdings in färberischer Hinsicht nicht zufriedenstellen konnten. Eine signifikante Verbesserung der Farbstabilität, insbesondere im Rotbereich, wurde durch den Austausch von p-Aminophenol durch die in der DE-OS 42 34 885, DE-OS 42 34 887 und EP-OS 0 375 977 beschriebenen 4,5-Diamino-pyrazole erzielt. Die Herstellung nach den dort beschriebenen Verfahren ist jedoch teilweise sehr aufwendig und erfordert zum Teil Rohstoffe, die teuer und nicht breit verfügbar sind. Weiterhin sind aus der EP-OS 0 740 931 in Position 3 des Pyrazols substituierte 4,5-Diamino-pyrazole bekannt. Diese Verbindungen müssen jedoch zum Teil nach einem aufwendigen Verfahren hergestellt werden, da insbesondere die C3-Komponenten nicht breit verfügbar sind. Die in der EP-OS 0 740 931 beschriebenen Pyrazole sind zudem in Bezug auf die erreichbare Farbpalette und die Farbsättigung nicht in jeder Hinsicht befriedigend.

Auch die WO-A- 99/11231 beschreibt 4,5-Diamino-pyrazolderivate für die oxidative Färbung von Keratinfasern.

Während die meisten Oxidationsfarbstoffe auf ungeschädigtem Haar kaum Schwächen zeigen, können sich gravierende Unterschiede auf geschädigtem Haar ergeben. Der Friseur kennt daher aus seiner Alltagspraxis das Problem, dass Farbstoffe nicht gleichmäßig auf das zu färbende Haar aufziehen. Während der Haaransatz in der Regel intakt ist, zeigen die Haarspitzen infolge von Witterungseinflüssen, häufigem Waschen und Kämmen im Laufe der Zeit eine Schädigung, die vom Haaransatz zur Haarspitze graduell zunimmt. Beim Färben solcher Haare kann infolge der ungleichmäßigen Haarbeschaffenheit zwischen Ansatz und Spitzen ein ungleichmäßiges Färbeergebnis erhalten werden. Ein weiteres Problem besteht darin, dass beim Waschen gefärbter Haare die Farbstoffe aus den stärker geschädigten Haarpartien, je nach dem Grad der Haarschädigung, stärker ausgewaschen werden als aus ungeschädigten Haarpartien, was nach einigen Haarwäschen immer deutlicher sichtbar werden kann.

Es bestand daher weiterhin ein grosser Bedarf nach zur Abdeckung des Rotbereichs bei oxidativen Färbesystemen geeigneten Farbstoff-Vorstufen, welche neben einem guten Aufziehverhalten über eine erheblich verbesserte Farbstabilität gegenüber Shampoonieren auf den verschiedensten Haarqualitäten, insbesondere durch Dauerwellen oder Blondieren geschädigtem Haar, verfügen.

Es wurde nun gefunden, dass die vorstehend genannte Aufgabe durch spezielle verbrückte Pyrazole in hervorragender Weise erfüllt wird.

Gegenstand der vorliegenden Erfindung sind daher 4,5-Diamino-pyrazole der Formel (I) oder dessen Salze mit organischen oder anorganischen Säuren,

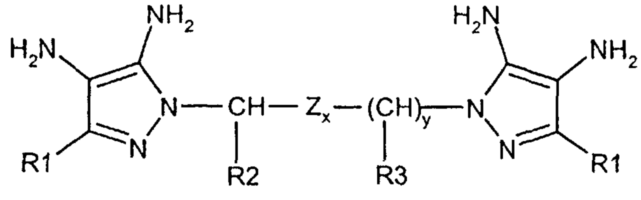

(I)

worin

**R1** Wasserstoff, eine geradkettige oder verzweigte C1-C6-Alkylgruppe, eine C1-C4-Hydroxyalkylgruppe, eine C1-C4-Aminoalkylgruppe, eine C1-C8-Alkylaminogruppe, eine Di(C1-C8)-alkylaminogruppe, eine C1-C4-Alkylamino-(C1-C4)alkylgruppe oder eine Di(C1-C4)-alkylamino-(C1-C4)-alkylgruppe, eine Arylgruppe oder eine Heteroarylgruppe darstellt;
**R2 und R3** gleich oder verschieden sein können und Wasserstoff, eine geradkettige oder verzweigte C1-C6-Alkylgruppe, eine Arylgruppe, eine Heteroarylgruppe, eine Carbonsäuregruppe, eine Carbonsäureestergruppe, eine unsubstituierte oder substituierte Carbonsäureamidgruppe, eine Hydroxygruppe oder eine C1-C4-Hydroxyalkylgruppe darstellen, oder aber R2 und R3 gemeinsam eine (gegebenenfalls substituierte) C1-C6-Alkylengruppe bilden;
**Z** gleich einem, gegebenenfalls durch ein Heteroatom (beispielsweise ein Stickstoff-, Sauerstoff- oder Schwefel-Atom) unterbrochenen C1-C10-Alkyl- Diradikal, einem, gegebenenfalls ein- oder zweifach benzokondensierten und/oder mit einer Hydroxygruppe oder (C1-C6)-Alkylgruppe substituierten, aromatischen oder heteroaromatischen Diradikal, oder einem Diradikal der Formel **-Ar-(Alk)$_n$-Ar-** ist, wobei gilt **Ar** gleich einem (gegebenenfalls substituierten) Arylenrest oder Heteroarylenrest (insbesondere Phenylenrest oder Pyridylenrest), **Alk** gleich einer -$CH_2$-Gruppe und **n** gleich einer ganzen Zahl von 0 bis 6; und
**x** und **y** unabhängig voneinander jeweils gleich 0 oder 1 sind.

Als geeignete Verbindungen der Formel (I) können beispielsweise genannt werden:

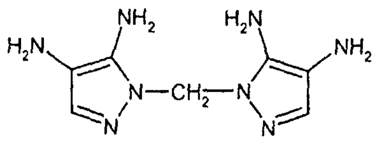

(I-a)

Bis-(4,5-diamino-pyrazol-1-yl)-methan

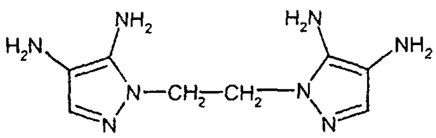

(I-b)

1,2-Bis-(4,5-diamino-pyrazol-1-yl)-ethan

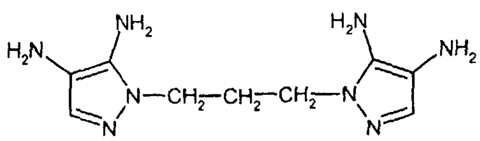

(I-c)

1,3-Bis-(4,5-diamino-pyrazol-1-yl)-propan

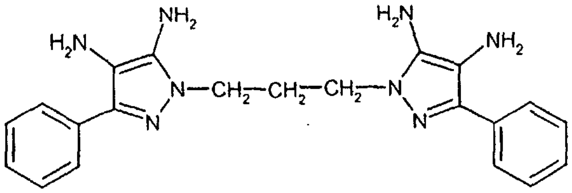

(I-d)

1,3-Bis-(4,5-diamino-3-phenyl-pyrazol-1-yl)-propan

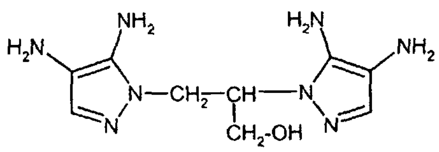

(I-e)

2,3-Bis-(4,5-diamino-pyrazol-1-yl)-propan-1-ol

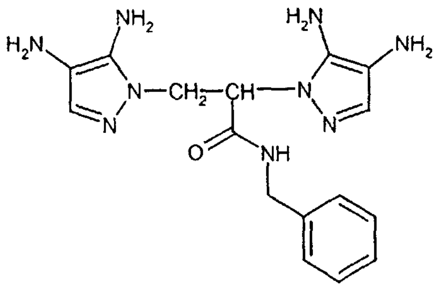

(I-f)

N-Benzyl-2,3-bis-(4,5-diamino-pyrazol-1-yl)-propionamid

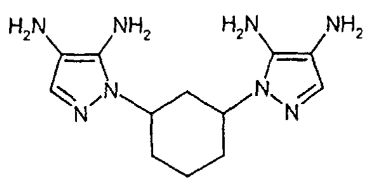

(I-g)

1,3-Bis-(4,5-diamino-pyrazol-1-yl)-cyclohexan

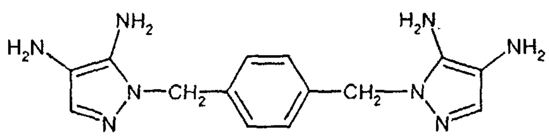

(I-h)

1,4-Bis-(4,5-diamino-pyrazol-1-yl-methyl)-benzol

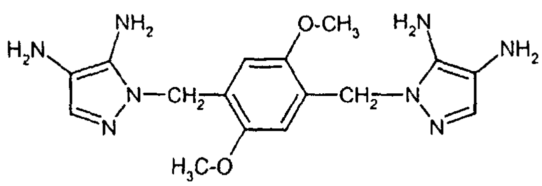

(I-i)

1,4-Bis-(4,5-diamino-pyrazol-1-yl-methyl)-2,5-dimethoxy-benzol

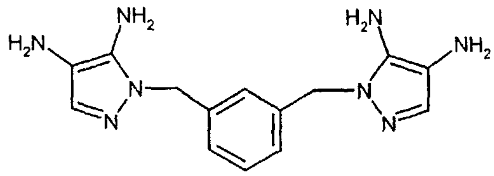

(I-j)

1,3-Bis-(4,5-diamino-pyrazol-1-yl-methyl)-benzol

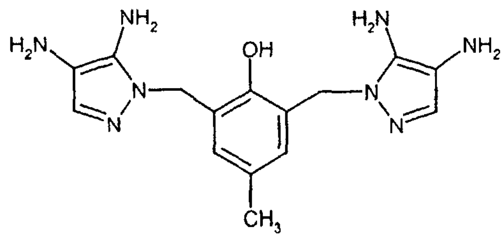

(I-k)

2,6-Bis-(4,5-diamino-pyrazol-1-yl-methyl)-4-methyl-phenol

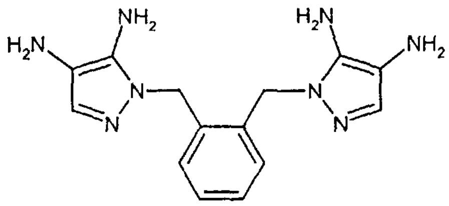

(I-l)

1,2-Bis -(4,5-diamino-pyrazol-1-yl-methyl)-benzol

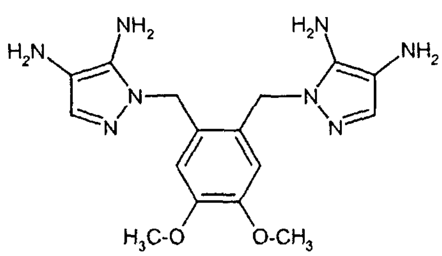

(I-m)

1,2-Bis -(4,5-diamino-pyrazol-1-yl-methyl)-4,5-dimethoxy-benzol

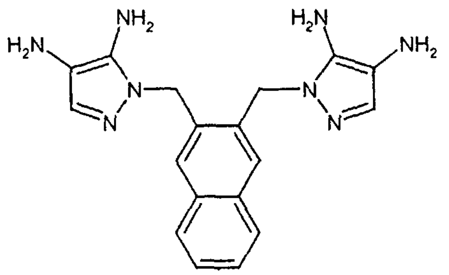

(I-n)

2,3-Bis-(4,5-diamino-pyrazol-1-yl-methyl)-naphthalin

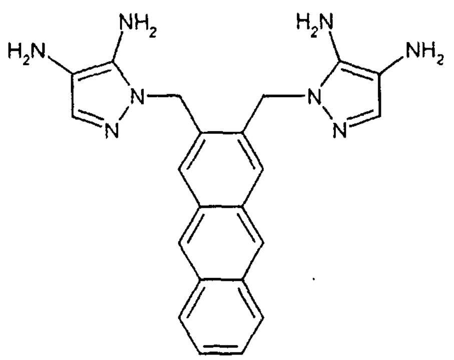

(I-o)

2,3-Bis-(4,5-diamino-pyrazol-1-yl-methyl)-anthracen

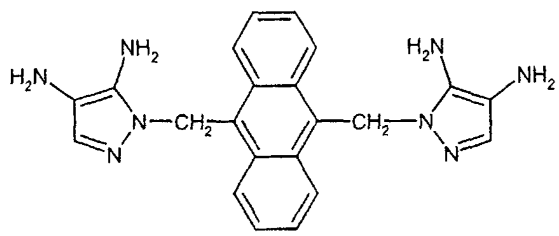

(I-p)

9,10-Bis-(4,5-diamino-pyrazol-1-yl-methyl)-anthracen

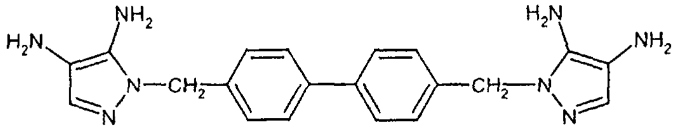

(I-q)

4,4'-Bis-(4,5-diamino-pyrazol-1-yl-methyl)-biphenyl

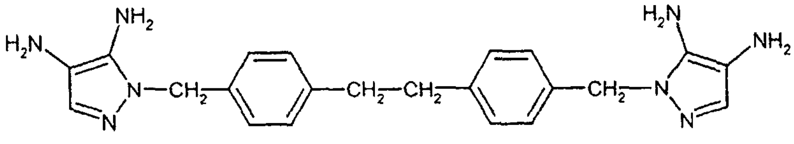

(I-r)

1,2-Bis-[4-(4,5-diamino-pyrazol-1-yl-methyl)-phenyl]-ethan

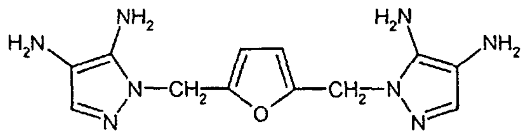

(I-s)

2,5-Bis-(4,5-diamino-pyrazol-1-yl-methyl)-furan

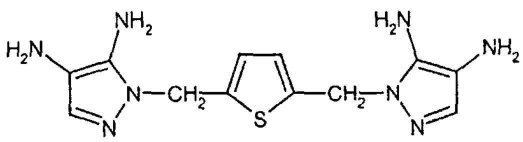

(I-t)

2,5-Bis-(4,5-diamino-pyrazol-1-yl-methyl)-thiophen

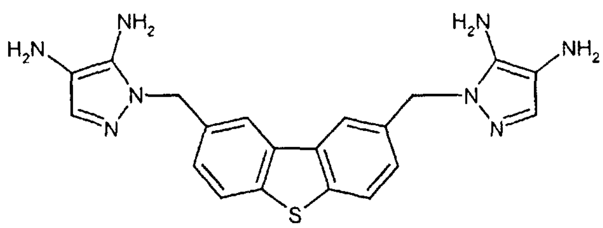

(I-u)

2,8-Bis-(4,5-diamino-pyrazol-1-yl-methyl)-dibenzothiophen

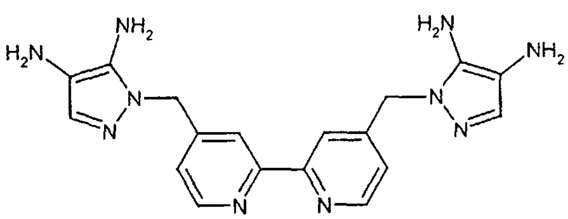

(I-v)

4,4'-Bis-(4,5-diamino-pyrazol-1-yl-methyl)-[2,2']bipyridyl

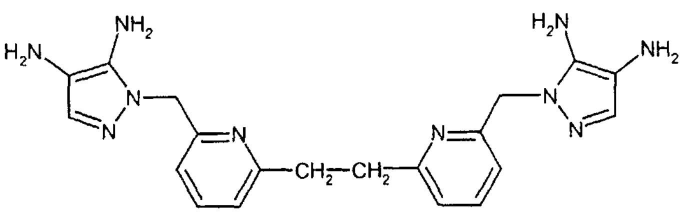

(I-w)

1,2-Bis-[6-(4,5-diamino-pyrazol-1-yl-methyl)-pyridin-2-yl]-ethan

Bevorzugt sind Verbindungen der allgemeinen Formel (I), in denen **R1** gleich Wasserstoff, einer Methylgruppe, einer Phenylgruppe, einer Thienylgruppe oder einer Furylgruppe ist; **R2** und **R3** unabhängig voneinander Wasserstoff,

eine Phenylgruppe, eine Carbonsäureamidgruppe oder eine Hydroxymethylgruppe darstellen; **Z** ein unsubstituiertes Alkylen-Diradikal, Phenylen-Diradikal oder Heteroarylen-Diradikal darstellt; und **x** und **y** unabhängig voneinander jeweils gleich 0 oder 1 sind.

Besonders bevorzugt sind Verbindungen, in denen die Reste **R1** bis **R3** gleich Wasserstoff sind; **Z** ein unsubstituiertes Alkylen-Diradikal, Phenylen-Diradikal oder Heteroarylen-Diradikal darstellt; und **x** und **y** unabhängig voneinander jeweils gleich 0 oder 1 sind.

Die Verbindungen der Formel (I) können nach verschiedenen Methoden hergestellt werden.

Eine Synthesemöglichkeit ist in Schema 1 dargestellt. Zunächst werden zwei Äquivalente 3,5-Dibrom-4-nitropyrazol mit einem Dihalogenid verbrückt. Nach der Umwandlung in die entsprechenden 5-Benzylaminoverbindungen und anschliessender katalytischer Hydrierung erhält man die 4,5-Diamino-pyrazole der allgemeinen Formel (I).

Schema 1

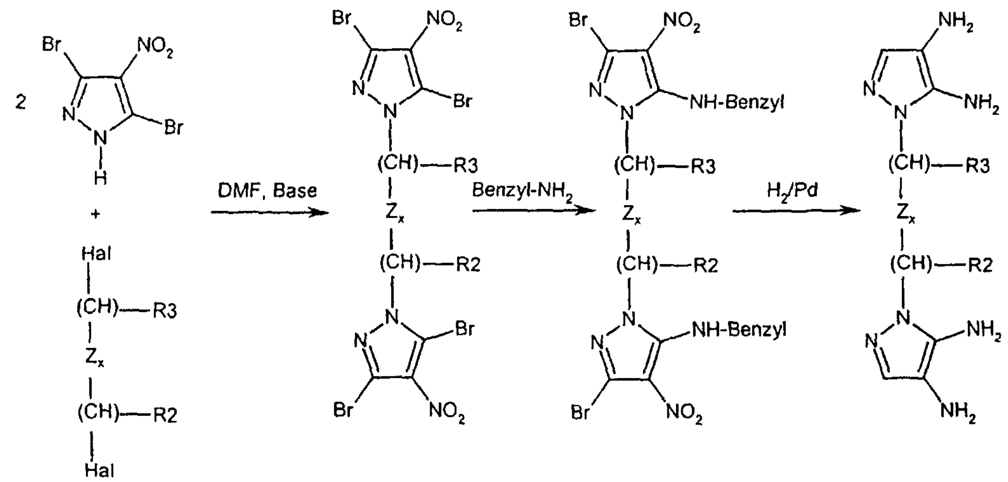


In bestimmten Fällen kann es jedoch von präparativem Vorteil sein, in der in Schema 2 skizzierten Weise, zunächst Cyanoethylhydrazin mit Dialdehyden oder Diketonen zu den entsprechenden Dihydrazonen umzusetzen und die erhaltenen Dihydrazone anschliessend basenkatalysiert zu den verbrückten 5-Aminopyrazolen zu zyklisieren, wobei die Herstellung dieser Zwischenstufen beispielsweise in Anlehnung an die in der FR-A 1 403 372 genannte Vorschrift erfolgt. Die anschliessende Einführung einer zweiten Pyrazol-Aminogruppe in Position 4, die beispielsweise via Azokupplung oder Nitrosierung möglich ist, erlaubt ebenfalls den Zugang zu 4,5-Diamino-pyrazolen der allgemeinen Formel (I), wobei bei diesem Syntheseweg vorzugsweise von Aldehyden ausgegangen wird.

Schema 2

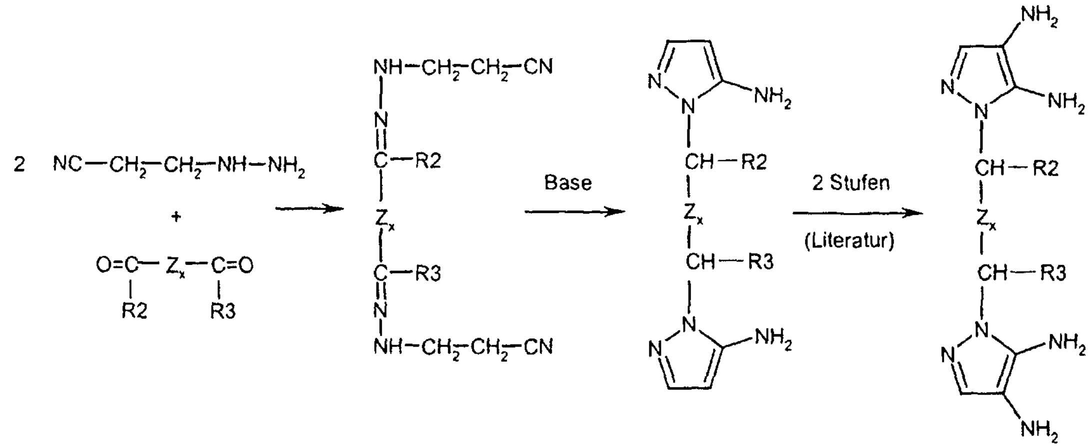


Die 5-Benzylamino-3-brom-Verbindungen aus Schema 1 können zur Einführung von Substituenten in Position 3 des Pyrazole verwendet werden. In idealer Weise kann dies nach Schema 3 beispielsweise durch Suzuki-Kupplung erfolgen, wobei auch weitere Kupplungsreaktionen denkbar sind.

Schema 3

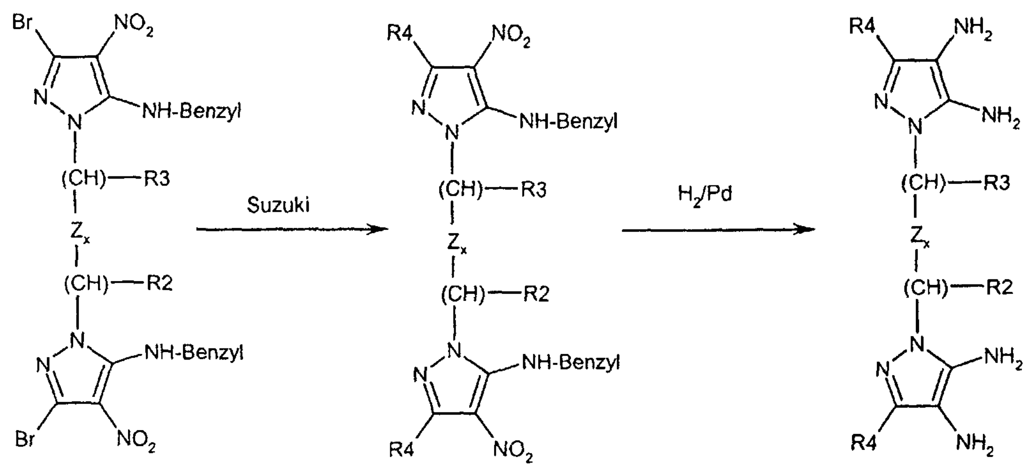


Wegen der Oxidationsempfindlichkeit der erfindungsgemäßen 4,5-Diamino-pyrazole werden die Verbindungen der Formel (I) aus Gründen der besseren Handhabbarkeit vorzugsweise nicht als freie Basen, sondern als Säureaddukte isoliert. Die so erhaltenen Salze sind weitgehend oxidationsunempfindlich. Als Säuren können hierbei anorganische oder organische Säuren eingesetzt werden, wobei Zitronensäure, Weinsäure und insbesondere Salzsäure und Schwefelsäure bevorzugt sind.

Die Verbindungen der Formel (I) eignen sich hervorragend als Farbstoff-Vorstufen im oxidativen System zum Färben von Keratinfasern. Obwohl sich die Verbindungen der Formel (I) insbesondere für die Verwendung zur Färbung von Keratinfasern, beispielsweise Wolle, Seide oder Haaren - insbesondere menschlicher Haare-, eignen, ist es prinzipiell auch möglich, mit diesen Verbindungen andere natürliche oder synthetische Fasern, beispielsweise Baumwolle oder Nylon 66, zu färben.

Ein weiterer Gegenstand der vorliegenden Erfindung ist daher ein Mittel zur oxidativen Färbung von Keratinfasern -insbesondere Haaren-, welches dadurch gekennzeichnet ist, dass es mindestens ein 4,5-Diaminopyrazol der allgemeinen Formel (I) oder dessen Salz mit organischen oder anorganischen Säuren, enthält.

Das 4,5-Diaminopyrazol der Formel (I) ist in dem erfindungsgemäßen Färbemittel in einer Menge von etwa

0,005 bis 20 Gewichtsprozent enthalten, wobei eine Menge von etwa 0,01 bis 10 Gewichtsprozent und insbesondere 0,1 bis 6 Gewichtsprozent bevorzugt ist.

Die Verbindungen der Formel (I) können sowohl alleine als auch in Kombination mit bekannten Entwicklersubstanzen und/oder Kupplersubstanzen, die üblicherweise in oxidativen Färbesystemen zur Färbung von Fasermaterialien Verwendung finden, eingesetzt werden.

Als geeignete Kupplersubstanzen können insbesondere genannt werden: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methylbenzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 2,4-Diamino-1-(3-methoxypropoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl) amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)-amino]-2-methyl-phenol, 3-[(2-Hydroxy-ethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 1,3-Dihydroxy-2,4-dimethyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 4-Hydroxy-indol, 5-Hydroxy-indol, 6-Hydroxy-indol, 7-Hydroxy-indol und 2,3-Indolindion, oder deren Salze.

Zur Herstellung von naturnahen Tönen und modischen Rottönen ist es besonders vorteilhaft, Verbindungen der Formel (I) in Kombination mit zusätzlichen Entwicklersubstanzen einzusetzen. Als Entwicklersubstanzen kommen p-Phenylendiamine, p-Aminophenole sowie weitere 4,5-Diamino-pyrazole oder deren Salze in Betracht.

Insbesondere sind die folgenden Entwicklersubstanzen zu nennen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methyl-phenyl)methyl]-1Hpyrazol, 1-[(4-Chlorphenyl)methyl]-4,5-diamino-1H-pyrazol, 4,5-Diamino-1-methyl-1H-pyrazol, 4,5-Diamino-3-methyl-1-phenyl-1H-pyrazol, 4,5-Diamino-1-(2-hydroxyethyl)-3-methyl-1H-pyrazol, 2-Amino-phenol, 2-Amino-6-methyl-phenol und 2-Amino-5-methyl-phenol oder deren Salze.

In dem erfindungsgemäßen Mittel sind die vorgenannten bekannten Entwicklersubstanzen und Kupplersubstanzen jeweils in einer Gesamtmenge von etwa 0,01 bis 20 Gewichtsprozent, vorzugsweise etwa 0,2 und 6 Gewichtsprozent, enthalten.

Die Verbindungen der Formel (I) können selbstverständlich auch in Kombination mit üblichen direktziehenden anionischen, kationischen, zwitterionischen oder nicht-ionischen Farbstoffen verwendet werden.

Zu den bevorzugten anionischen Farbstoffen zählen beispielsweise: 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Monound Disulfonsäure) (CI47005;D&C Yellow No. 10; Food Yellow No. 13, Acid Yellow No. 3), 5-Hydroxy-1-(4-sulfophenyl)-4-[(4-sulfophenyl)azo]-pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylamino-benzolsulfonsäure-natriumsalz (CI10385; Acid Orange No. 3), 4-[(2,4-Dihydroxyphenyl)azo]-benzolsulfonsäuremononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäure-dinatriumsalz (CI14720; Acid Red No. 14), 6-Hydroxy-5-[(4-sulfonaphth-1-yl)azo]-2,4-naphthalindisulfonsäure-trinatriumsalz (CI16255; Ponceau 4R; Acid Red No. 18), 3-Hydroxy-4-[(4-sulfonaphth-1-yl)azo]-2,7-naphthalin-disulfonsäuretrinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)-azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäuredinatriumsalz (CI45430;Acid Red No. 51 ), N-[6-(Diethylamino)-9-(2,4-disulfophenyl)-3H-xanthen-3-yliden]-N-ethylethanaminium-hydroxid, inneres Salz, Natriumsalz (CI45100; Acid Red No. 52), 8-[(4-(Phenylazo)-phenyl)azo]-7-naphthol-1,3-disulfonsäure-dinatriumsalz (CI27290; Acid Red No. 73), 2',4',5',7'-Tetrabrom-3',6'-dihydroxyspiro[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen)-3-on-dinatriumsalz (CI45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)-phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)-carbenium-inneres salz, Natriumsalz (2:1) (CI42045; Food Blue No. 3; Acid Blue No. 1), Bis[4-(diethylamino)phenyl](5-hydroxy-2,4-disulfophenyl)-carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäuredinatriumsalz (CI73015; Acid Blue No. 74), 9-(2-Carboxyphenyl)-3-[(2-methylphenyl)-amino]-6-[(2-methyl-4-sulfophenyl)amino]xanthyliuminneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalindisulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo)naphth-1-yl)azo]-1,7-naphthalindisulfonsäuretetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäure Natriumsalz Chrom-Komplex (Acid Red No. 195).

Zu den bevorzugten kationischen Farbstoffen zählen beispielsweise: 9-(Dimethylamino)-benzo[a]phenoxazin-7-ium-chlorid (CI51175; Basic Blue No. 6), Di[4-(diethylamino)phenyl][4-(ethylamino)naphthyl]carbeniumchlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)-phenyl][4-(methylamino)phenyl]carbenium-chlorid (CI42535; Basic Violet No. 1), Tris(4-amino-3-methylphenyl)-carbenium-chlorid (CI42520; Basic Violet No. 2), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphtholchlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitro-phenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxy-phenyl)azo)-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)-azo]-pyrazol-5-on-chlorid (CI12719; Basic Yellow No. 57) und Bis[4-(diethyl-amino)phenyl]phenylcarbenium-hydrogensulfat(1:1) (CI42040; Basic Green No. 1).

Als geeignete nichtionische Farbstoffe (insbesondere zum besseren Farbausgleich und zur Erzeugung von speziellen Nuancen können beispielsweise geannt werden: 1-Amino-2-[(2-hydroxyethyl)amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitro-phenol, 1-(2-Hydroxyethoxy)-3-methylamino-4-nitrobenzol, 2,3-(Dihydroxy-propoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No. 9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxy-ethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitro-benzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl) amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl) amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl) amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxy-ethyl)amino]-3-methylbenzol (HC Yellow No. 7), 2,6-Diamino-3-[(pyridin-3-yl)azo]-pyridin, 2-((4-(Acetylamino)phenyl)-azo)-4-methylphenol (CI11855; Disperse Yellow No. 3).

Aus der Gruppe der direktziehenden Farbstoffe besonders zu erwähnen sind 2-Amino-4,6-dinitrophenol, 2-Ethylamino-4,6-dinitrophenol, 2-[(2-Hydroxy-ethyl)amino]-4,6-dinitrophenol sowie Farbstoffe der allgemeinen Formel (II),

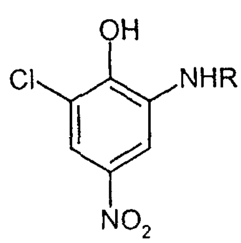

(II)

worin R Wasserstoff, Methyl, Ethyl oder Hydroxyethyl bedeutet.

Die Gesamtkonzentration an direktziehenden Farbstoffen beträgt in dem erfindungsgemäßen Mittel etwa 0,01 bis 10 Gewichtsprozent, bzw. etwa 0,1 bis 10 Gewichtsprozent, vorzugsweise etwa 0,1 bis 5 Gewichtsprozent.

Selbstverständlich können die Farbstoffe, sofern es Basen sind, auch in Form ihrer physiologisch verträglichen Salze mit organischen oder anorganischen Säuren, wie beispielsweise Salzsäure oder Schwefelsäure, beziehungsweise, sofern sie aromatische OH-Gruppen besitzen, in Form der Salze mit Basen, zum Beispiel als Alkaliphenolate, eingesetzt werden.

Die vorstehend beschriebenen erfindungsgemäßen Kombinationen der Verbindungen der Formel (I) mit oxidativen Haarfarbvorstufen und/oder direktziehenden Farbstoffen werden zur Färbung in einer geeigneten Farbträgermasse appliziert.

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch

eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem Oxidationsmittel unmittelbar vor der Anwendung hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12-prozentigen, vorzugsweise einer 3- bis 6-prozentigen, wässrigen Lösung, in Betracht. Das Gewichtsverhältnis zwischen Haarfärbemittel und Oxidationsmittel beträgt hierbei vorzugsweise etwa 5:1 bis 1:3, insbesondere 1:1 bis 1:2. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Es ist prinzipiell auch möglich, zur Oxidation der Farbstoffe anstelle der vorgenannten Oxidationsmittel Luftsauerstoff zu verwenden.

Der pH-Wert des gebrauchsfertigen erfindungsgemäßen Haarfärbemittels stellt sich bei der Mischung der Farbträgermasse (deren pH-Wert etwa gleich 6 bis 11,5 ist) mit dem meist sauer eingestellten Oxidationsmittel (dessen pH-Wert etwa gleich 2 bis 6,5 ist) auf einen pH-Wert ein, der durch die Alkalimengen in der Farbträgermasse und die Säuremengen im Oxidationsmittel sowie durch das Mischungsverhältnis bestimmt wird. Je nach Zusammensetzung kann das erfindungsgemäße Färbemittel schwach sauer, neutral oder alkalisch reagieren und im gebrauchsfertigen Zustand einen pH-Wert von etwa 3 bis 11, vorzugsweise etwa 5 bis 10, H aufweisen. Die basische Einstellung erfolgt hierbei vorzugsweise mit Ammoniak, wobei jedoch auch organische Amine, zum Beispiel 2-Amino-2-methyl-1-propanol, Tris(hydroxymethyl)amino-methan, Monoethanolamin und Triethanolamin, oder auch anorganische Basen wie Natriumhydroxid und Kaliumhydroxid Verwendung finden. Für eine pH-Wert-Einstellung im sauren Bereich kommen anorganische oder organische Säuren, zum Beispiel Phosphorsäure, Essigsäure, Milchsäure, Ascorbinsäure, Zitronensäure oder Weinsäure, in Betracht.

Anschliessend trägt man eine für die Haarfärbebehandlung ausreichende Menge, je nach Haarfülle, im allgemeinen etwa 60 bis 200 Gramm, dieses Gemisches auf das Haar auf und läßt das Gemisch bei etwa 15 bis 50 Grad Celsius, vorzugsweise 30 bis 40 Grad Celsius, etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an 4,5-Diamino-pyrazolen der Formel (I) ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, insbesondere im Bereich der modischen Rottöne. Hierbei zeichnen sich die Farbtöne durch ihre besondere Farbintensität und Leuchtkraft aus. Die sehr guten färberischen Eigenschaften der Färbemittel gemäß der vorliegenden Anmeldung zeigen sich insbesondere darin, dass diese Mittel auch unterschiedlich stark vorgeschädigtem Haar eine gleichmässige und haltbare Anfärbung ermöglichen.

Die nachfolgenden Beispiele sollen den Gegenstand der Erfindung näher erläutern, ohne ihn darauf zu beschränken.

**Beispiele**

**Beispiel 1: Herstellung von 1,2-Bis-(4,5-diamino-pyrazol-1-yl)-ethantetrahydrochlorid**

**Stufe 1.1: Glyoxal-bis-(2-(2-cyanoethyl))-hydrazon**

Zu 170,2 g 2-Cyanoethylhydrazin werden unter leichter Kühlung 145,1 g Glyoxal 40% so zugetropft, dass eine Temperatur von 40 °C nicht überschritten wird. Anschliessend wird die Reaktionsmischung etwa 1 Stunde lang

bei Raumtemperatur weitergerührt, wobei das Hydrazon auskristallisiert. Das Produkt wird abgesaugt, gut abgepresst und getrocknet.
Ausbeute : 170,5 g beige Kristalle
Schmelzpunkt: 96-97 °C.
$^1$H-NMR (DMSO-$d_6$): δ= 2,66 ppm (t, $^3J_{HH}$ = 10,75 Hz, 2H); 3,29 ppm (dt, $^3J_{HH}$ = 10,75 Hz, $^3J_{HH}$ = 8 Hz, 2H); 7,32 ppm (s, 2H); 7,33 ppm (t, $^3J_{HH}$ = 8 Hz, 2H).

**Stufe 1.2: 1,2-Bis-(5-amino-pyrazol-1-yl)-ethan**

17,3 g Hydrazon aus Stufe 1.1 werden in 150 ml Butoxyethanol zusammen mit 10,1 g Kalium-tert-butylat auf 110 °C erhitzt. Nach 1 Stunde lässt man abkühlen und saugt die ausgefallenen Kristalle ab. Nachwaschen mit wenig Butoxyethanol und Trocknen ergeben 18,2 g kupferfarbene Blättchen mit metallischem Oberflächenglanz.
Schmelzpunkt: 208-209 °C.
$^1$H-NMR (DMSO-$d_6$): δ= 4,17 ppm (s, 4H); 5,06 ppm (s, 4H); 7,27 ppm (d, $^3J_{HH}$ = 3 Hz, 2H); 7,10 ppm (d, $^3J_{HH}$ = 3 Hz, 2H).

**Stufe 1.3: 1,2-Bis-(5-amino-4-nitroso-pyrazol-1-yl)-ethan∗2HCl**

10 g 1,2-Bis-(5-amino-pyrazol-1-yl)-ethan aus Stufe 1.2 werden unter Kühlung und auf 0 bis 5 °C in 150 ml Tetrahydrofuran mit 29,6 g konz. Salzsäure versetzt. Zu dieser Mischung tropft man innerhalb von 30 Minuten 12,2 g Isopentylnitrit und lässt 3 Stunden lang weiterrühren. Man saugt die gelbe Suspension ab und setzt ohne weitere Reinigung zum Endprodukt um.

**Stufe 1.4: 1,2-Bis-(4,5-diamino-pyrazol-1-yl)-ethan∗4HCl**

Das Rohprodukt aus Stufe 1.3 wird in 150 ml Wasser unter leicht erhöhtem Wasserstoffdruck an 1,5 g Palladium (10% auf Aktivkohle) hydriert. Nach 6 Stunden wird der Katalysator abfiltriert und das Filtrat unter vermindertem Druck auf etwa 50 ml eingeengt. Nach der Zugabe von 100 ml konzentrierter Salzsäure rührt man noch 30 Minuten lang im Eisbad, saugt sodann den Rückstand ab und trocknet im Vakuum.
Ausbeute: 11,1 g eines beigen Produkts
Schmelzpunkt: >250 °C.
$^1$H-NMR (DMSO-$d_6$): δ= 4,27 ppm (s, 4H); 6,80 ppm (s, breit, 8H); 7,32 ppm (s, 2H); 9,95 ppm (s, breit, 4H). FAB-MS: 223 $[M+H]^+$

**Beispiel 2: Herstellung von 1,2-Bis-(4,5-diamino-pyrazol-1-yl)-ethansulfat**

3,23 g 1,2-Bis-(5-amino-4-nitroso-pyrazol-1-yl)-ethan-dihydrochlorid aus Bespiel 1, Stufe 1.3, werden in 50 ml Wasser mit Ammoniaklösung neutralisiert. Der ausgefallene Niederschlag wird abgesaugt und in 30 ml 2-Methoxyethanol unter leicht erhöhtem Wasserstoffdruck an 0,2 g Palladium (10% auf Aktivkohle) hydriert. Nach 6 Stunden wird der Katalysator abfiltriert, das Filtrat im Eisbad gekühlt und unter Rühren tropfenweise mit 1 g konzentrierter Schwefelsäure versetzt. Das ausgefallene Produkt wird abgesaugt und aus 30 ml Ethanol/Wasser 1:1 umkristallisiert. Absaugen und Trocknen im Vakuum ergeben 1,7 g eines beigen Produkts.
Schmelzpunkt: >250°C.

| Elementaranalyse: ($C_8H_{14}N_8$ x $H_2SO_4$; M = 320,33) | | | | |
|---|---|---|---|---|
| Unter Berücksichtigung von 1,44% Kristallwasser ergeben sich folgende Werte: | | | | |
| | %C | %H | %N | %S |
| ber. | 29,56 | 5,12 | 34,48 | 9,87 |
| gef. | 29,40 | 4,90 | 33,60 | 9,90 |

**Beispiel 3: 1,4-Bis-(4,5-diamino-pyrazol-1-yl-methyl)-benzol∗4HCl**

**Stufe 3.1: 3-(N'-{4-[(2-Cyano-ethyl)-hydrazonomethyl]-benzyliden}hydrazino)-propionitril**

Zu einer Suspension von 102 g Terephthaldehyd in 700 ml Methanol tropft man innerhalb von 20 Minuten

129,4 g 2-Cyanoethylhydrazin. Die Temperatur steigt hierbei auf etwa 50 °C an, wobei allmählich eine orange-rote Lösung entsteht. Nach ungefähr weiteren 10 Minuten setzt Kristallisation ein. Man rührt noch 30 Minuten lang in einem Eisbad weiter und saugt den ausgefallenen Niederschlag ab. Nachwaschen mit wenig kaltem Methanol und Trocknen im Vakuum führt zu 187 g eines beigefarbenen Produktes
Schmelzpunkt: 111-113 °C.

| Elementaranalyse: ($C_{14}H_{16}N_6$; M = 268,32) | | | |
|---|---|---|---|
| | %C | %H | %N |
| ber. | 62,67 | 6,01 | 31,32 |
| gef. | 62,40 | 6,08 | 31,48 |

**Stufe 3.2: 1,4-Bis-(5-amino-pyrazol-1-yl-methyl)-benzol**

Das in Stufe 3.1 erhaltene 3-(N'-{4-[(2-Cyano-ethyl)-hydrazonomethyl]-benzyliden}-hydrazino)-propionitril wird in 750 ml Butanol / 45g Kalium-tert-butylat auf 75 °C erwärmt. Nach 2 Stunden lässt man auf Raumtemperatur abkühlen und filtriert den ausgefallenen Niederschlag ab. Nachwaschen mit wenig kaltem Butanol und Trocknen im Vakuum ergibt 91,1 g hellbraunes Produkt.
Schmelzpunkt: 204-206°C.
$^1$H-NMR (DMSO-$d_6$): δ= 5,07 ppm (s, 4H); 5,21 ppm (s, 4H); 5,29 ppm (d, $^3J_{HH}$ = 3.1 Hz, 2H); 7,06 ppm (d, $^3J_{HH}$ = 3,1 Hz, 2H); 7.07 ppm (s, 4H).

| Elementaranalyse: ($C_{14}H_{16}N_6$; M = 268.32) | | | |
|---|---|---|---|
| | %C | %H | %N |
| ber. | 62,67 | 6,01 | 31,32 |
| gef. | 62,61 | 6,13 | 31,49 |

**Stufe 3.3: 1,4-Bis-(5-amino-4-nitroso-pyrazol-1-yl-methyl)-benzol*2HCl**

2,2 g 1,4-Bis-(5-amino-pyrazol-1-yl-methyl)-benzol aus Stufe 3.2 werden in 40 ml Ethanol suspendiert, mit 1,9 g konzentrierter Salzsäure versetzt und anschließend im Eisbad innerhalb von 15 min tropfenweise mit 2,2 g Isoamylnitrit versetzt. Nach weiterem 3-stündigem Rühren im Eisbad wird abgesaugt und getrocknet. Man erhält 2,7 g eines rötlichen Feststoffs, der als Rohprodukt weiterverarbeitet wird.

**Stufe 3.4: 1,4-Bis-(4,5-diamino-pyrazol-1-yl-methyl)-benzol*4HCl**

2,7 g 1,4-Bis-(5-amino-4-nitroso-pyrazol-1-yl-methyl)-benzoldihydrochlorid aus Stufe 3.3 werden in 40 ml Ethanol unter leicht erhöhtem Wasserstoff-druck an 0,3 g Palladium (10% auf Aktivkohle) hydriert. Nach 5 Stunden wird der Katalysator abfiltriert. Zum Filtrat gibt man 20 ml ethanolische Salzsäure, rührt 30 Minuten lang im Eisbad, saugt ab und trocknet im Vakuum.
Ausbeute: 2,5 g eines beigen Produkts.
Die Titration mit 0,1n Natronlauge bestätigt das Vorliegen der Substanz als Tetrahydrochlorid.
$^1$H-NMR (DMSO-$d_6$): δ= 5,15 ppm (s, 4H); 5,7 ppm (s breit, NH und Wasser); 7,14 ppm (s, 4H); 7,29 ppm (s, 2H); 9,90 ppm (s breit, 4H).
FAB-MS: 299 $[M+H]^+$

**Beispiel 4: 1,3-Bis-(4,5-diamino-pyrazol-1-yl-methyl)-benzol*4HCl**

**Stufe 4.1: 1,3-Bis-(3,5-dibrom-4-nitro-pyrazol-1-yl-methyl)-benzol**

In 40 ml Dimethylformamid werden 13,54 g 3,5-Dibrom-4-nitropyrazol, 11,1 g Kaliumcarbonat, 0,8 g Kaliumiodid und 6,6 g α,α'-Dibrom-m-xylol auf 100 °C erhitzt. Nach 50 Minuten lässt man die Reaktionsmischung abkühlen und gießt auf 400 ml Wasser. Man filtriert den ausgefallenen Niederschlag ab, wäscht mit Wasser nach und trocknet im Vakuum.
Ausbeute: 13,9 g gelbliches Produkt.
$^1$H-NMR (DMSO-$d_6$): δ= 5,53 ppm (s, 4H); 7,03 ppm (s, 1H); 7,24 ppm (d, $^3J_{HH}$ = 12.85 Hz, 2H); 7,43 ppm (t, $^3J_{HH}$ =

12.85 Hz, 1H).

**Stufe 4.2: 1,3-Bis-(5-benzylamino-3-brom-4-nitro-pyrazol-1-ylmethyl)-benzol**

13,9 g 1,3-Bis-(3,5-dibrom-4-nitro-pyrazol-1-yl-methyl)-benzol aus Stufe 4.1 werden zusammen mit 6,5 g Benzylamin in 130 ml n-Propanol 2 Stunden lang auf 95 °C erhitzt. Anschliessend kühlt man ab und gießt auf 400 ml Wasser. Der ausgefallene Niederschlag wird abgesaugt und aus 150 ml Acetonitril umkristallisiert.
Ausbeute: 6,6 g.
$^1$H-NMR (DMSO-$d_6$): δ= 4,51 ppm (d, $^3J_{HH}$ = 11 Hz, 4H); 5,22 ppm (s, 4H); 6,75 (s, 1H); 7,04 ppm (d, $^3J_{HH}$ = 11 Hz, 2H); 7,16 ppm (d, $^3J_{HH}$ = 11 Hz, 2H); 7,2 - 7,4 ppm (m, 6H); 8,01 ppm (t, $^3J_{HH}$ = 11 Hz, 2H).

**Stufe 4.3: 1,3-Bis-(4,5-diamino-pyrazol-1-yl-methy)-benzol∗4HCl**

1,7 g 1,3-Bis-(5-benzylamino-3-brom-4-nitro-pyrazol-1-yl-methyl)-benzol aus Stufe 4.2 werden in 30 ml 2-Methoxyethanol 3 Stunden lang an 0,2 g Palladium (10% auf Aktivkohle) bei einer Temperatur von 50 °C und 5 bar Wasserstoffdruck hydriert. Anschliessend wird abgekühlt, filtriert und zur
Trockene eingedampft. Der Rückstand wird in 20 ml ethanolischer Salzsäure aufgenommen, wobei Kristallisation einsetzt. Absaugen und Trocknen im Vakuum ergibt 1,5 g beiges Produkt.
Die Titration mit 0,1 n Natronlauge bestätigt das Vorliegen der Substanz als Tetrahydrochlorid.
FAB-MS: 299 $[M+H]^+$

**Beispiel 5: 1,3-Bis-(4,5-diamino-pyrazol-1-yl)-propan∗4HCl**

**Stufe 5.1: 1,3-Bis-(3,5-dibrom-4-nitro-pyrazol-1-yl)-propan**

In 60 ml Dimethylformamid werden 27,01 g 3,5-Dibrom-4-nitropyrazol, 16,4 g Natriumacetat und 11 g 1,3-Dibrompropan auf 100°C erhitzt. Nach 2 Stunden wird die Reaktionsmischung abgekühlt und der Reaktionsansatz unter Rühren auf 500 ml Eiswasser gegossen. Das ausgefallene Produkt wird abgesaugt, mit Wasser gut nachgewaschen und im Vakuum bei
60 °C getrocknet.
Ausbeute: 25,6 g beiges Produkt
Schmelzpunkt: 194-198 °C.

| Elementaranalyse: ($C_9H_6Br_4N_6O_4$; M = 581,82) | | | |
|---|---|---|---|
| | %C | %H | %N |
| ber. | 18,58 | 1,04 | 14,44 |
| gef. | 18,43 | 1,05 | 14,20 |

**Stufe 5.2: 1,3-Bis-(5-benzylamino-3-brom-4-nitro-pyrazol-1-yl)-propan**

11,6 g 1,3-Bis-(3,5-dibrom-4-nitro-pyrazol-1-yl)-propan aus Stufe 5.1 und 6,6 g Benzylamin werden in 180 ml n-Propanol 1 Stunde lang auf 100 °C erhitzt. Anschliessend kühlt man ab und gießt auf 1 Liter Wasser. Der ausgefallene gelbliche Niederschlag wird abgesaugt, mit Wasser nachgewaschen und im Vakuum bei 60 °C getrocknet.
Ausbeute: 10,9 g gelbliches Produkt
Schmelzpunkt: 148-151°C.

| Elementaranalyse: ($C_{23}H_{22}Br_2N_8O_4$; M = 634,30) | | | |
|---|---|---|---|
| | %C | %H | %N |
| ber. | 43,55 | 3,50 | 17,67 |
| gef. | 43,85 | 3,54 | 17,38 |

**Stufe 5.3: 1,3-Bis-(4,5-diamino-pyrazol-1-yl)-propan∗4HCl**

3,1 g 1,3-Bis-(5-benzylamino-3-brom-4-nitro-pyrazol-1-yl)-propan aus Stufe 5.2 werden in 80 ml 2-Methoxyethanol 6 Stunden lang an 0,4 g Palladium (10% auf Aktivkohle) bei Raumtemperatur und 5 bar Wasserstoffdruck

hydriert. Anschliessend wird filtriert und annähernd zur Trockene eingedampft. Rühren des Rückstandes in einer Mischung aus 20 ml Ethanol und 20 ml 2n-Salzsäure, filtrieren des ausgefallenen Niederschlags und trocknen im Vakuum ergibt 0,2 g beiges Produkt.
Die Titration mit 0,1 n Natronlauge bestätigt das Vorliegen der Substanz als Tetrahydrochlorid.
$^1$H-NMR (DMSO-$d_6$): δ= 2,05 ppm (t verbreitert, $^3J_{HH}$ = 11 Hz, 2H); 3,96 ppm (t, $^3J_{HH}$ = 11 Hz, 4H); 4,01 (NH und Wasser); 7,28 ppm (s, 2H); 9,82 ppm (s breit, 4H).
FAB-MS: 237 [M+H]$^+$

**Beispiel 6: 1,3-Bis-(4,5-diamino-3-phenyl-pyrazol-1-yl)-propan*4HCl**

**Stufe 6.1: : 1,3-Bis-(5-benzylamino-4-nitro-3-phenyl-pyrazol-1-yl)-propan**

Unter Stickstoff werden 3,7 g 1,3-Bis-(5-benzylamino-3-brom-4-nitropyrazol-1-yl)-propan aus Stufe 5.2 in 40 ml 1,2-Dimethoxyethan vorgelegt, mit 3,8 g Phenylborsäure, 1,2 g Tetrakis(triphenylphosphin)-palladium(0) und der Lösung von 8,3 g Kaliumcarbonat in 30 ml Wasser versetzt und die Reaktionsmischung anschliessend unter Rückfluß erhitzt. Nach 5 Stunden wird heiß über Kieselgur (Hyflo Super-Gel der Fa. Celite) filtriert und das Filtrat im Eisbad gekühlt. Der ausgefallene Niederschlag wird abfiltriert, mit Wasser gewaschen und aus 50 ml Ethanol umkristallisiert. Nach dem Trocknen im Vakuum bei 60°C erhält man 1,5 g gelbliches Produkt
Schmelzpunkt: 142-144 °C.
$^1$H-NMR (DMSO-$d_6$): δ= 2,35 ppm (t verbreitert, $^3J_{HH}$ = 11 Hz, 2H); 4,22 ppm (t, $^3J_{HH}$ = 11 Hz, 4H); 4,74 (d, $^3J_{HH}$ = 11 Hz, 4H); 7,1-7,4 ppm (m, 20H); 7,70 ppm (d, $^3J_{HH}$ = 11 Hz, 2H).

**Stufe 6.2: 1,3-Bis-(4,5-diamino-3-phenyl-pyrazol-1-yl)-propan*4HCl**

1,5 g 1,3-Bis-(5-benzylamino-4-nitro-3-phenyl-pyrazol-1-yl)-propan aus Stufe 6.1 werden in 50 ml Essigsäure 3 Stunden lang an 1,5 g Palladium (10% auf Aktivkohle) bei 5 bar Wasserstoffdruck 15 Stunden hydriert. Anschliessend wird filtriert und annähernd zur Trockene eingedampft. Der Rückstand wird in 20 ml Ethanol und 10 ml konzentrierter Salzsäure 3 Stunden lang unter Rückfluß erhitzt. Beim Abkühlen im Eisbad scheidet sich ein viskoser Niederschlag ab. Dekantieren der überstehenden Lösung und Aufnehmen des Rückstandes in 20 ml ethanolischer Salzsäure ergibt 0,5 g beiges Produkt.
$^1$H-NMR (DMSO-$d_6$): δ= 2,18 ppm (t verbreitert, $^3J_{HH}$ = 11 Hz, 2H); 3,50 ppm (s breit, NH und Wasser); 4,02 ppm (t, $^3J_{HH}$ = 11 Hz, 4H); 7,2-7,7 ppm (m, 10H); 10,10 ppm (s breit, 4H).
FAB-MS: 389 [M+H]$^+$

**Beispiel 7: Oxidationshaarfärbemittel, basisch**

| | |
|---|---|
| 0,30 g | Ascorbinsäure |
| 0,40 g | Natriumsulfit |
| 10,00 g | Natriumlaurylethersulfat, 28%ige wässrige Lösung |
| 7,85 g | Ethanol |
| 0,92 g | Pyrazol der Formel (I) gemäß Beispiel 1 |
| y g | Kuppler nach Tabelle 1 |
| 9,10 g | Ammoniak, 25 %ige wässrige Lösung |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und die erhaltene gebrauchsfertige Färbelösung in der erforderlichen Menge auf gebleichte Haare aufgetragen. Nach einer Einwirkzeit von 30 Minuten bei 40 °C wird das Haar mit einem Shampoo gewaschen, mit Wasser ausgespült und getrocknet. Die erhaltenen Farbnuancen und Farbintensitäten sind in Tabelle 1 zusammengefasst.

Tabelle 1

| Bsp. | [g] | Kupplersubstanz | Farbton | Intensität |
|---|---|---|---|---|
| **7.1** | 0,28 g | Resorcin | rosé | o |
| **7.2** | 0,31 g | 1,3-Dihydroxy-2-methyl-benzol | rosé | o |
| **7.3** | 0,35 g | 1,3-Dihydroxy-2,4-dimethyl-benzol | rotblond | + |
| **7.4** | 0,27 g | 3-Aminophenol | kupfergold | ++ |
| **7.5** | 0,31 g | 5-Amino-2-methylphenol | orange-rot | ++ |
| **7.6** | 0,64 g | 5-((2-Hydroxyethyl)amino)-2-methoxyanilin-dihydrochlorid | schwarzviolett | ++ |
| **7.7** | 0,56 g | 6-Amino-3,4-dihydro-2H-1,4-benzoxazin-dihydrochlorid | rotviolett | ++ |
| **7.8** | 0,52 g | 3,4-Dihydro-6-hydroxy-1,4(2H)-benzoxazin-sulfat(2:1)-hydrat(2:1) | aschblond | ++ |
| **7.9** | 0,59 g | 1,3-Diamino-4-methoxybenzol-sulfat | weinrot | ++ |
| **7.10** | 0,60 g | 1,3-Diamino-4-(2-hydroxy-ethoxy)-benzol-dihydrochlorid | dunkelviolett | ++ |
| **7.11** | 1,08 g | 1,3-Di(2,4-diaminophenoxy)-propan-tetrahydrochlorid | dunkelweinrot | ++ |
| **7.12** | 0,36 g | 1-Chlor-2,4-dihydroxybenzol | himbeer | ++ |
| **7.13** | 0,57 g | 3-Amino-6-methoxy-2-(methylamino)-pyridin-hydrochlorid | blauviolett | ++ |
| **7.14** | 0,35 g | 5-Hydroxy-1,3-benzodioxol | cognac | + |
| **7.15** | 0,43 g | 5-Amino-1,3-benzodioxol-hydrochlorid | himbeer | + |
| **7.16** | 0,54 g | 5-((2-Hydroxyethyl)amino)-1,3-benzodioxol-hydrochlorid | kaminrot | ++ |
| **7.17** | 0,36 g | 1-Naphthol | violett | ++ |
| **7.18** | 0,44 g | 4-Methoxy-1-naphthol | violett | + |
| **7.19** | 0,50 g | 1-Acetoxy-2-methylnaphthol | rotviolett | ++ |
| **7.20** | 0,61 g | 3,5-Diamino-2,6-dimethoxy-pyridin-dihydrochlorid | weinrot | ++ |
| **7.21** | 0,40 g | 1,7-Dihydroxynaphthalin | violett | + |
| **7.22** | 0,45 g | 3-Dimethylamino-phenyl-harnstoff | stahlblau | ++ |
| **7.23** | 0,33 g | 4-Hydroxyindol | rotviolett | ++ |
| **7.24** | 0,39 g | 3-Amino-2-chlor-6-methyl-phenol | kupferrot | ++ |
| **7.25** | 0,56 g | 2-Chlor-5-((2,2,2-trifluor-ethyl)amino)-phenol | weinrot | ++ |
| **7.26** | 0,45 g | 3-Amino-2,4-dichlorphenol | bordeaux | ++ |
| **7.27** | 0,36 g | 5-Amino-2-chlor-phenol | glutrot | ++ |
| (o) = mittel, (+) = stark, (++) = sehr stark | | | | |

**Beispiel 8: Oxidationshaarfärbemittel, basisch**

| | |
|---|---|
| 0,30 g | Ascorbinsäure |
| 0,40 g | Natriumsulfit |
| 10,00 g | Natriumlaurylethersulfat, 28%ige wässrige Lösung |
| 7,85 g | Ethanol |
| 1,11 g | Pyrazol der Formel (I) gemäß Beispiel 3 |
| y g | Kuppler nach Tabelle 2 |
| 9,10 g | Ammoniak, 25 %ige wässrige Lösung |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40 °C wird das Haar mit einem Shampoo gewaschen, mit Wasser ausgespült und getrocknet. Die erhaltenen Farbnuancen und Farbintensitäten sind in Tabelle 2 zusammengefasst.

Tabelle 2

| Bsp. | [g] | Kupplersubstanz | Farbton | Intensität |
|---|---|---|---|---|
| **8.1** | 0,56 g | Resorcin | himbeer | + |
| **8.2** | 0,54 g | 3-Aminophenol | weinrot | ++ |
| **8.3** | 0,62 g | 5-Amino-2-methylphenol | kupfer-rot | ++ |
| **8.4** | 1,28 g | 5-((2-Hydroxyethyl)amino)-2-methoxyanilin-dihydrochlorid | schwarz, violette Reflexe | ++ |
| **8.5** | 1,20 g | 1,3-Diamino-4-(2-hydroxy-ethoxy)-benzol-dihydrochlorid | rotschwarz | ++ |
| **8.6** | 2,16 g | 1,3-Di(2,4-diaminophenoxy)-propan-tetrahydrochlorid | schwarz, rote Reflexe | ++ |
| **8.7** | 0,90 g | 3-Dimethylamino-phenyl-harnstoff | dunkelblau | ++ |
| (o) = mittel, (+) = stark, (++) = sehr stark | | | | |

**Beispiele 9-11: Cremeförmiges Oxidationshaarfärbemittel, basisch**

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| x g | Farbstoffe nach Tabelle 3 |
| 4,50 g | Ammoniak, 25%ige wässrige Lösung |
| ad 100,00 g | Wasser, entmineralisiert |

Der pH-Wert der Cremes liegt zwischen 10,1 und 10,5.

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40 °C wird das Haar mit einem Shampoo gewaschen, mit Wasser ausgespült und getrocknet.
Die erhaltenen Nuancen sind Tabelle 3 zu entnehmen.

Tabelle 3

| Farbstoff \ Beispiel | 9 | 10 | 11 |
|---|---|---|---|
| Pyrazol nach Beispiel 2 | 4,26 g | | |
| Pyrazol nach Beispiel 4 | | | 4,26 g |
| Pyrazol nach Beispiel 5 | | 2,22 g | |
| 3-Amino-phenol | | | 0,22 g |
| 4-Amino-3-methyl-phenol | 0,01 g | | 0,10 g |
| 5-Amino-2-methyl-phenol | 0,39 g | | |
| 3-Amino-2-chlor-6-methyl-phenol | 1,01 g | | 0,30 g |
| 1,3-Dihydroxybenzol | | 0,56 g | |
| 2-Amino-6-chlor-4-nitro-phenol*HCl | | 0,51 g | |
| 2-Chlor-6-(ethylamino)-4-nitro-phenol | | 0,05 g | |
| 1,4-Diamino-2-methyl-benzol-sulfat | | | 0,31 g |
| 1,4-Diamino-2-(2-hydroxyethyl)-benzol-sulfat | | | 0,20 g |
| N-(3-(Dimethylamino)-phenyl)-harnstoff | | | 0,34 g |
| **erhaltene Nuance** | **leuchtend glutrot** | **leuchtend rotgold** | **schwarz mit rotvioletten Reflexen** |

**Beispiel 12: Gelförmiges Oxidationshaarfärbemittel**

| | |
|---|---|
| 15,00 g | Ölsäure |
| 3,00 g | Glycerin |
| 7,00 g | Isopropanol |
| 0,50 g | Ascorbinsäure |
| 0,40 g | Natriumsulfit |
| 0,40 g | Natriumhydroxid |
| 10,00 g | Ammoniak, 25%ige wässrige Lösung |
| 1,00 g | Pyrazol nach Beispiel 2 |

(fortgesetzt)

| | |
|---:|---|
| 0,31 g | 1,4-Diamino-2-methyl-benzol-sulfat |
| 0,20 g | 1,4-Diamino-2-(2-hydroxyethyl)-benzol-sulfat |
| 0,10 g | 4-Amino-3-methyl-phenol |
| 0,46 g | 1,3-Diamino-4-(2-hydroxyethoxy)-benzol-dihydrochlorid |
| 0,24 g | 5-Amino-2-methylphenol |
| 0,21 g | 3-Amino-phenol |
| ad 100,00 g | Wasser, entmineralisiert |

Das Gel hat einen pH-Wert von 10,8.

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf zu 50% ergrautem Humanhaar aufgetragen. Nach einer Einwirkzeit von 30 Minuten bei 40°C wird das Haar mit einem Shampoo gewaschen, mit Wasser gespült und getrocknet. Das Haar ist schwarz mit einem Auberginereflex gefärbt.

**Beispiel 13: Cremeförmiges Oxidationshaarfärbemittel, sauer**

| | |
|---:|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| 0,56 g | Pyrazol nach Beispiel 3 |
| 0,67 g | Pyrazol nach Beispiel 6 |
| 0,25 g | 5-((2-Hydroxyethyl)amino)-1,3-benzodioxol-hydrochlorid |
| 0,18 g | 3-Amino-2-chlor-6-methyl-phenol |
| 0,22 g | 6-Amino-3,4-dihydro-2H-1,4-benzoxazin-dihydrochlorid |
| ad 100,00 g | Wasser, entmineralisiert |

Der pH-Wert der Creme wird mit Ammoniak 25% auf 6,6 eingestellt.

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf unterschiedliche Haare (siehe Tabelle 4) aufgetragen. Nach einer Einwirkzeit von 30 Minuten bei 40°C wird das Haar mit einem Farbpflegeshampoo gewaschen, mit Wasser gespült und getrocknet.

Tabelle 4

| **Haarsträhnen ungefärbt** | **gefärbt** |
|---|---|
| Yak-Haar, weiß | aubergine |
| zu 50% ergrautes Humanhaar | aubergine, Grauanteile vollständig abgedeckt |
| Humanhaar, mittelbraun | aubergine dunkel |

**Beispiel 14: Vergleichsversuche zur Farbstabilität**

| **Verwendete Farbträgermasse:** | |
|---:|---|
| 0,30 g | Ascorbinsäure |
| 0,40 g | Natriumsulfit |

(fortgesetzt)

| **Verwendete Farbträgermasse:** | |
|---|---|
| 10,00 g | Natriumlaurylethersulfat, 28%ige wässrige Lösung |
| 7,85 g | Ethanol |
| x g | Pyrazol der Formel (I) gemäß Tabelle 5 bis 8 |
| 0,31 g | 5-Amino-2-methylphenol |
| 9,10 g | Ammoniak, 25 %ig |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf die nachfolgend beschriebenen Haarsträhnen aufgetragen. Nach einer Einwirkzeit von 30 Minuten bei 40 °C werden die Haare mit einem Shampoo gewaschen, mit Wasser ausgespült und getrocknet. Danach werden die L*a*b*-Werte der Referenzsträhnen gemessen. Anschliessend werden die Strähnen je dreimal je 1 Minute shampooniert, getrocknent und erneut nach L*a*b* vermessen.
Die Ergebnisse der Versuche sind in den Tabellen 5 bis 11 zusammengefasst.
In den Vergleichsversuchen verwendete Haartypen:

**a) Humanhaar, mittelblond**
Das Humanhaar wurde zuvor nicht gefärbt, blondiert oder gewellt.
**b) Humanhaar, blondiert**
Mittelblondes Humanhaar wurde 15 Minuten lang bei 40°C mit einem handelsüblichen Blondiermittel behandelt, mit Wasser ausgespült und sodann getrocknet.
**c) Humanhaar, dauergewellt**
Mittelblondes Humanhaar wurde 20 Minuten lang bei 40°C mit einem handelsüblichen Dauerwellmittel behandelt, dann fixiert, mit einer üblichen Pflegespülung nachbehandelt und getrocknet.

In den folgenden Tabellen 5 bis 8 stehen die ΔE-Werte für Farbveränderungen im L*a*b*-System und werden nach der Gleichung

$$\Delta E = \sqrt{(L_i - L_o)^2 + (a_i - a_o)^2 + (b_i - b_o)^2}$$

berechnet. Darin stellen $L_0$, $a_0$ und $b_0$ Meßwerte vor und $L_i$, $a_i$ und $b_i$ die entsprechenden Werte nach den Waschversuchen dar (zur Erläuterung: je höher der errechnete ΔE-Wert ist, desto höher ist der Farbverlust bzw. die Farbtonänderung).
Die Berechnungen der Mittelwerte erfolgte mit Microsoft Excel 97 SR-2, Funktion "Mittelwert".
Alle in der vorliegenden Anmeldung enthaltenen Prozentangaben stellen, soweit nicht anders angegeben, Gewichtsprozente dar.

Tabelle 5

| (erfindungsgemäß; mit x=0,92 g Pyrazol aus Beispiel 1) | | | | | |
|---|---|---|---|---|---|
| **Haartyp** | | **L*** | **a*** | **b*** | **ΔE** |
| (a) unbehandeltes Humanhaar | gefärbt | 31,81 | 20,69 | 20,18 | |
| | gewaschen | 32,63 | 20,94 | 20,92 | 1,13 |
| (b) blondiertes Humanhaar | gefärbt | 36,97 | 34,53 | 30,90 | |
| | gewaschen | 38,41 | 34,23 | 31,72 | 1,68 |
| (c) dauergewelltes Humanhaar | gefärbt | 26,72 | 20,70 | 16,39 | |
| | gewaschen | 27,09 | 21,57 | 15,43 | 1,35 |
| | | | Mittelwert ΔE: 1,39 | | |

Tabelle 6

| (Stand der Technik; mit x=0,46 g 4,5-Diamino-1-methyl-pyrazol gemäß EP-OS 0 375 977) | | | | | |
|---|---|---|---|---|---|
| **Haartyp** | | **L*** | **a*** | **b*** | **ΔE** |
| (a) unbehandeltes Humanhaar | gefärbt | 34,73 | 19,64 | 24,15 | |
| | gewaschen | 37,73 | 19,16 | 25,68 | 3,40 |
| (b) blondiertes Humanhaar | gefärbt | 47,49 | 35,19 | 41,24 | |
| | gewaschen | 51,97 | 32,22 | 40,77 | 5,40 |
| (c) dauergewelltes Humanhaar | gefärbt | 33,10 | 18,20 | 22,61 | |
| | gewaschen | 37,04 | 16,64 | 22,72 | 4,24 |
| Mittelwert ΔE: 4,35 | | | | | |

Tabelle 7

| (Stand der Technik; mit x=0,57 g 4,5-Diamino-3-methyl-1-(2-hydroxyethyl)- pyrazol gemäß Beispiel 8 der EP-OS 0 740) | | | | | |
|---|---|---|---|---|---|
| **Haartyp** | | **L*** | **a*** | **b*** | **ΔE** |
| (a) unbehandeltes Humanhaar | gefärbt | 26,59 | 21,78 | 9,72 | |
| | gewaschen | 29,98 | 21,74 | 11,05 | 3,64 |
| (b) blondiertes Humanhaar | gefärb | 31,05 | 34,56 | 10,86 | |
| | gewaschen | 37,78 | 33,26 | 13,74 | 7,43 |
| (c) dauergewelltes Humanhaar | gefärbt | 23,41 | 22,10 | 8,52 | |
| | gewaschen | 30,37 | 20,76 | 12,49 | 8,12 |
| Mittelwert ΔE: 6,40 | | | | | |

Tabelle 8

| (Stand der Technik; mit x=0,53 g 4,5-Diamino-1-(isopropyl)-pyrazol gemäß DE-OS 42 34 885) | | | | | |
|---|---|---|---|---|---|
| **Haartyp** | | **L*** | **a*** | **b*** | **ΔE** |
| (a) unbehandeltes Humanhaar | gefärbt | 32,57 | 28,71 | 20,52 | |
| | gewaschen | 33,76 | 28,59 | 21,08 | 1,32 |
| (b) blondiertes Humanhaar | gefärbt | 36,63 | 48,27 | 30,21 | |
| | gewaschen | 40,29 | 47,46 | 32,36 | 4,32 |
| (c) dauergewelltes Humanhaar | gefärbt | 26,64 | 28,98 | 15,94 | |
| | gewaschen | 31,97 | 23,72 | 15,79 | 7,49 |
| Mittelwert ΔE: 4,38 | | | | | |

Die vorliegenden Vergleichsversuche belegen eindeutig die gegenüber dem Stand der Technik verbesserte Auswaschfestigkeit der erfindungsgemäßen Pyrazole, insbesondere bei vorgeschädigtem Haar.

## Patentansprüche

1. 4,5-Diamino-pyrazole der Formel (I) oder deren Salze mit organischen oder anorganischen Säuren,

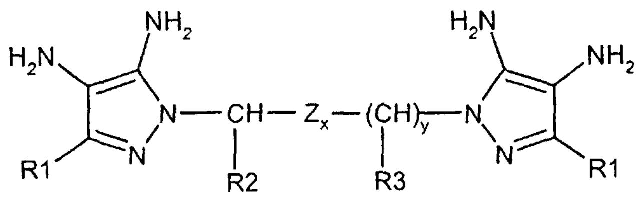

(I),

worin

**R1** Wasserstoff, eine geradkettige oder verzweigte C1-C6-Alkylgruppe, eine C1-C4-Hydroxyalkylgruppe, eine C1-C4-Aminoalkylgruppe, eine C1-C8-Alkylaminogruppe, eine Di(C1-C8)-alkylaminogruppe, eine C1-C4-Alkylamino-(C1-C4)alkylgruppe oder eine Di(C1-C4)-alkylamino-(C1-C4)-alkylgruppe, eine Arylgruppe oder eine Heteroarylgruppe darstellt;

**R2 und R3** gleich oder verschieden sein können und Wasserstoff, eine geradkettige oder verzweigte C1-C6-Alkylgruppe, eine Arylgruppe, eine Heteroarylgruppe, eine Carbonsäuregruppe, eine Carbonsäureestergruppe, eine unsubstituierte oder substituierte Carbonsäureamidgruppe, eine Hydroxygruppe oder eine C1-C4-Hydroxyalkylgruppe darstellen, oder aber R2 und R3 gemeinsam eine (gegebenenfalls substituierte) C1-C6-Alkylengruppe bilden;

**Z** gleich einem, gegebenenfalls durch ein Heteroatom unterbrochenen C1-C10-Alkyl- Diradikal, einem, gegebenenfalls ein- oder zweifach benzokondensierten und/oder mit einer Hydroxygruppe oder (C1-C6)-Alkylgruppe substituierten, aromatischen oder heteroaromatischen Diradikal, oder einem Diradikal der Formel **$-Ar-(Alk)_n-Ar-$** ist, wobei gilt **Ar** gleich einem (gegebenenfalls substituierten) Arylenrest oder Heteroarylenrest, **Alk** gleich einer $-CH_2$-Gruppe und **n** gleich einer ganzen Zahl von 0 bis 6; und

**x** und **y** unabhängig voneinander jeweils gleich 0 oder 1 sind.

**2.** 4,5-Diamino-pyrazol gemäß Anspruch 1, **dadurch gekennzeichnet, dass** es ausgewählt ist aus Bis-(4,5-diamino-pyrazol-1-yl)-methan, 1,2-Bis-(4,5-diamino-pyrazol-1-yl)-ethan, 1,3-Bis-(4,5-diamino-pyrazol-1-yl)-propan, 1,3-Bis-(4,5-diamino-3-phenyl-pyrazol-1-yl)-propan, 2,3-Bis-(4,5-diamino-pyrazol-1-yl)-propan-1-ol, N-Benzyl-2,3-bis-(4,5-diaminopyrazol-1-yl)-propionamid, 1,3-Bis-(4,5-diamino-pyrazol-1-yl)-cyclohexan, 1,4-Bis-(4,5-diamino-pyrazol-1-yl-methyl)-benzol, 1,4-Bis-(4,5-diaminopyrazol-1-yl-methyl)-2,5-dimethoxy-benzol, 1,3-Bis-(4,5-diamino-pyrazol-1-yl-methyl)-benzol, 2,6-Bis-(4,5-diamino-pyrazol-1-yl-methyl)-4-methylphenol, 1,2-Bis -(4,5-diamino-pyrazol-1-yl-methyl)-benzol, 1,2-Bis-(4,5-diamino-pyrazol-1-yl-methyl)-4,5-dimethoxy-benzol, 2,3-Bis-(4,5-diaminopyrazol-1-yl-methyl)-naphthalin, 2,3-Bis-(4,5-diamino-pyrazol-1-yl-methyl)-anthracen, 9,10-Bis-(4,5-diamino-pyrazol-1-yl-methyl)-anthracen, 4,4'-Bis-(4,5-diamino-pyrazol-1-yl-methyl)-biphenyl, 1,2-Bis-[4-(4,5-diaminopyrazol-1-yl-methyl)-phenyl]-ethan, 2,5-Bis-(4,5-diamino-pyrazol-1-ylmethyl)-furan, 2,5-Bis-(4,5-diamino-pyrazol-1-yl-methyl)-thiophen, 2,8-Bis-(4,5-diamino-pyrazol-1-yl-methyl)-dibenzothiophen, 4,4'-Bis-(4,5-diaminopyrazol-1-yl-methyl)-[2,2']bipyridyl und 1,2-Bis-[6-(4,5-diamino-pyrazol-1-yl-methyl)-pyridin-2-yl]-ethan oder deren Salzen mit organischen oder anorganischen Säuren.

**3.** 4,5-Diamino-pyrazol nach Anspruch 1, **dadurch gekennzeichnet, dass R1** gleich Wasserstoff, einer Methylgruppe, einer Phenylgruppe, einer Thienylgruppe oder einer Furylgruppe ist; **R2** und **R3** unabhängig voneinander Wasserstoff, eine Phenylgruppe, eine Carbonsäureamidgruppe oder eine Hydroxymethylgruppe darstellen; **Z** ein unsubstituiertes Alkylen-Diradikal, Phenylen-Diradikal oder Heteroarylen-Diradikal darstellt; und x und y unabhängig voneinander jeweils gleich 0 oder 1 sind.

**4.** 4,5-Diamino-pyrazol nach Anspruch 3, **dadurch gekennzeichnet, dass R1** bis **R3** gleich Wasserstoff sind.

**5.** 4,5-Diamino-pyrazol nach einem der Ansprüche 1 bis 4, **dadurch gekennzeichnet, dass** es ein Salz der Schwefelsäure, Salzsäure, Zitronensäure oder Weinsäure ist.

**6.** Mittel zur oxidativen Färbung von Keratinfasern, **dadurch gekennzeichnet, dass** es mindestens ein 4,5-Diaminopyrazol nach einem der Ansprüche 1 bis 5 enthält.

**7.** Mittel nach Anspruch 6, **dadurch gekennzeichnet, dass** es das 4,5-Diaminopyrazol in einer Menge von 0,005 bis 20 Gewichtsprozent enthält.

**8.** Mittel nach Anspruch 6 oder 7, **dadurch gekennzeichnet, dass** es zusätzlich weitere Entwicklersubstanzen und/oder Kupplersubstanzen in einer Gesamtmenge von jeweils 0,01 bis 20 Gewichtsprozent enthält.

**9.** Mittel nach einem der Ansprüche 6 oder 8, **dadurch gekennzeichnet, dass** es zusätzlich 0,01 bis 10 Gewichtsprozent direktziehende Farbstoffe in enthält.

**10.** Mittel nach einem der Ansprüche 6 bis 9, **dadurch gekennzeichnet, dass** die Farbträgermasse mit dem Oxidationsmittel in einem Gewichtsverhältnis von 5:1 bis 1:3 vermischt wird.

**11.** Mittel nach Anspruch 10, **dadurch gekennzeichnet, dass** das gebrauchsfertige Oxidationsfärbemittel einen pH-Wert von 3 bis 11 aufweist.

## Claims

**1.** 4,5-Diaminopyrazoles of the formula (I) or salts thereof with organic or inorganic acids,

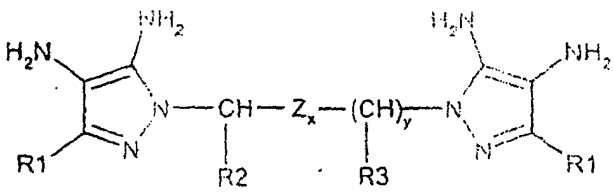

(I),

in which

**R1** is hydrogen, a straight-chain or branched C1-C6-alkyl group, a C1-C4-hydroxyalkyl group, a C1-C4-alkylamino group, a C1-C8-alkylamino group, a di(C1-C8)alkylamino group, a C1-C4-alkylamino-(C1-C4)alkyl group or a di (C1-C4)alkylamino-(C1-C4)alkyl group, an aryl group or a heteroaryl group;

**R2 and R3** may be identical or different and are hydrogen, a straight-chain or branched C1-C6-alkyl group, an aryl group, a heteroaryl group, a carboxylic acid group, a carboxylic ester group, an unsubstituted or substituted carboxamide group, a hydroxyl group or a C1-C4-hydroxyalkyl group, or else R2 and R3 together form an (optionally substituted) C1-C6-alkylene group;

**Z** is a C1-C10-alkyl diradical optionally interrupted by a heteroatom, an aromatic or heteroaromatic diradical optionally mono- or di-benzofused and/or substituted by a hydroxyl group or (C1-C6)alkyl group, or a diradical of the formula $-Ar-(Alk)_n-Ar-$, where **Ar** is an (optionally substituted) arylene radical or heteroarylene radical, **Alk** is a $-CH_2$ group and **n** is an integer from 0 to 6; and

**X** and **y**, independently of one another, are each 0 or 1.

**2.** 4,5-Diaminopyrazole according to Claim 1, **characterized in that** it is chosen from bis(4,5-diaminopyrazol-1-yl) methane, 1,2-bis(4,5-diamino-pyrazol-1-yl)ethane, 1,3-bis(4,5-diaminopyrazol-1-yl)propane, 1,3-bis(4,5-diamino-3-phenylpyrazol-1-yl)propane, 2,3-bis(4,5-diaminopyrazol-1-yl)propan-1-ol, N-benzyl-2,3-bis(4,5-diamino-pyrazol-1-yl)propionamide, 1,3-bis(4,5-diamino-pyrazol-1-yl)cyclohexane, 1,4-bis(4,5-diamino-pyrazol-1-ylmethyl) benzene, 1,4-bis(4,5-diamino-pyrazol-1-ylmethyl)-2,5-dimethoxybenzene, 1,3-bis(4,5-diaminopyrazol-1-ylmethyl)benzene 2,6-bis(4,5-diaminopyrazol-1-ylmethyl)-4-methylphenol, 1,2-bis(4,5-diaminopyrazol-1-ylmethyl) benzene, 1,2-bis(4,5-diaminopyrazol-1-ylmethyl)-4,5-dimethoxybenzene, 2,3-bis(4,5-diaminopyrazol-1-ylmethyl) naphthalene, 2,3-bis(4,5-diaminopyrazol-1-ylmethyl)anthracene, 9,10-bis(4,5-diaminopyrazol-1-ylmethyl)anthracene, 4,4'-bis(4,5-diaminopyrazol-1-ylmethyl)biphenyl, 1,2-bis[4-(4,5-diaminopyrazol-1-ylmethyl)phenyl]ethane, 2,5-bis(4,5-diaminopyrazol-1-ylmethyl)furan, 2,5-bis(4,5-diaminopyrazol-1-ylmethyl)thiophene, 2,8-bis(4,5-diaminopyrazol-1-ylmethyl)dibenzothio-phene, 4,4'-bis(4,5-diaminopyrazol-1-ylmethyl)-[2.2']bipyridyl and 1,2-bis [6-(4,5-diaminopyrazol-1-ylmethyl)pyridin-2-yl]ethane or salts thereof with organic or inorganic acids.

**3.** 4,5-Diaminopyrazole according to Claim 1, **characterized in that R1** is hydrogen, a methyl group, a phenyl group, a thienyl group or a furyl group; **R2** and **R3**, independently of one another, are hydrogen, a phenyl group, a carboxamide group or a hydroxymethyl group; **Z** is an unsubstituted alkylene diradical, phenylene diradical or heteroarylene diradical; and **x** and **y**, independently of one another, are in each case 0 or 1.

**4.** 4,5-Diaminopyrazole according to Claim 3, **characterized in that R1** to **R3** are hydrogen.

**5.** 4,5-Diaminopyrazole according to one of Claims 1 to 4, **characterized in that** it is a salt of sulphuric acid, hydrochloric acid, citric acid or tartaric acid.

**6.** Agent for the oxidative colouring of keratin fibres, **characterized in that** it comprises at least one 4,5-diaminopyrazole according to one of Claims 1 to 5.

**7.** Agent according to Claim 6, **characterized in that** it comprises the 4,5-diaminopyrazole in an amount of from 0.005 to 20 per cent by weight.

**8.** Agent according to Claim 6 or 7, **characterized in that** it additionally comprises further developer substances and/or coupler substances in a total amount of in each case 0.01 to 20 per cent by weight.

**9.** Agent according to one of Claims 6 or 8, **characterized in that** it additionally comprises 0.01 to 10 per cent by weight of direct dyes.

**10.** Agent according to one of Claims 6 to 9, **characterized in that** the colour carrier mass is mixed with the oxidizing agent in a weight ratio of from 5:1 to 1:3.

**11.** Agent according to Claim 10, **characterized in that** the ready-to-use oxidation colorant has a pH of from 3 to 11.

**Revendications**

**1.** 4,5-diamino-pyrazoles de formule (I) ou leurs sels avec des acides organiques ou minéraux,

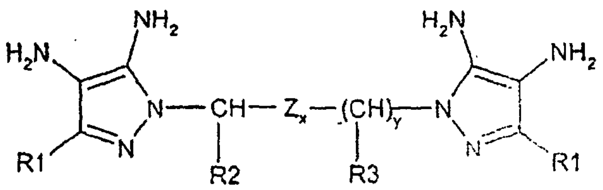

(I)

formule dans laquelle
R1 représente un atome d'hydrogène, un groupe alkyle en $C_1$-$C_6$ à chaîne droite ou ramifiée, un groupe hydroxyalkyle en $C_1$-$C_4$, un groupe aminoalkyle en $C_1$-$C_4$, un groupe alkyl($C_1$-$C_8$)amino, un groupe dialkyl($C_1$-$C_8$)amino, un groupe al kyl ($C_1$-$C_4$)amino-alkyle($C_1$-$C_4$) ou un groupe dialkyl($C_1$-$C_4$)amino-alkyle($C_1$-$C_4$), un groupe aryle ou un groupe hétéroaryle ;
R2 et R3 peuvent être identiques ou différents et représentent un atome d'hydrogène, un groupe alkyle en $C_1$-$C_6$ à chaîne droite ou ramifiée, un groupe aryle, un groupe hétéroaryle, un groupe carboxy, un groupe ester d'acide carboxylique, un groupe carbamoyle substitué ou non substitué, un groupe hydroxy ou un groupe hydroxyalkyle en $C_1$-$C_4$, ou bien R2 et R3 forment ensemble un groupe alkylène en $C_1$-$C_6$ (éventuellement substitué) ;
Z représente un diradical alkyle en $C_1$-$C_{10}$ éventuellement interrompu par un hétéroatome, un diradical aromatique ou hétéroaromatique éventuellement une ou deux fois condensé à un noyau benzénique et/ou substitué par un groupe hydroxy ou un groupe alkyle en $C_1$-$C_6$, ou un diradical de formule -Ar-$(Alk)_n$-Ar-, Ar représentant un radical arylène ou hétéroarylène (éventuellement substitué), Alk représentant un groupe $-CH_2-$ et n étant un nombre entier allant de 0 à 6 ; et
x et y valent chacun, indépendamment l'un de l'autre, 0 ou 1.

**2.** 4,5-diamino-pyrazole selon la revendication 1, **caractérisé en ce qu'**il est choisi parmi le bis(4,5-diamino-pyrazol-1-yl)méthane, le 1,2-bis(4,5-diamino-pyrazol-1-yl)éthane, le 1,3-bis(4,5-diamino-pyrazol-1-yl)propane, le 1,3-bis (4,5-diamino-3-phényl-pyrazol-1-yl)propane, le 2,3-bis(4,5-diamino-pyrazol-1-yl)propan-1-ol, le N-benzyl-2,3-bis (4,5-diamino-pyrazol-1-yl)-propionamide, le 1,3-bis'(4,5-diamino-pyrazol-1-yl)-cyclohexane, le 1,4-bis(4,5-diamino-pyrazol-1-yl-méthyl)benzène, le 1,4-bis(4,5-diamino-pyrazol-1-yl-méthyl)-2,5-diméthoxybenzène, le 1,3-bis

(4,5-diamino-pyrazol-1-yl-méthyl)benzène, le 2,6-bis(4,5-diamino-pyrazol-1-yl-méthyl)-4-méthylphénol, le 1,2-bis(4,5-diamino-pyrazol-1-yl-méthyl)benzène, le 1,2-bis(4,5-diamino-pyrazol-1-yl-méthyl)-4,5-diméthoxybenzène, le 2,3-bis(4,5-diamino-pyrazol-1-yl-méthyl)naphtalène, le 2,3-bis(4,5-diamino-pyrazol-1-yl-méthyl)anthracène, le 9,10-bis(4,5-diamino-pyrazol-1-yl-méthyl)anthracène, le 4,4'-bis(4,5-diamino-pyrazol-1-yl-méthyl)biphényle, le 1,2-bis[4-(4,5-diamino-pyrazol-1-yl-méthyl)phényl]-éthane, le 2,5-bis(4,5-diamino-pyrazol-2-yl-méthyl)-furanne, le 2,5-bis(4,5-diamino-pyrazol-1-yl-méthyl)-thiophène, le 2,8-bis(4,5-diamino-pyrazol-1-yl-méthyl)-dibenzothiophène, le 4,4'-bis(4,5-diamino-pyrazol-1-yl-méthyl)-[2,2']bipyridyle et le 1,2-bis[6-(4,5-diamino-pyrazol-1-yl-méthyl)pyridin-2-yl]éthane ou leurs sels avec des acides organiques ou minéraux.

**3.** 4,5-diamino-pyrazole selon la revendication 1, **caractérisé en ce que** R1 représente un atome d'hydrogène, un groupe méthyle, un groupe phényle, un groupe thiényle ou un groupe furyle ; R2 et R3 représentent, indépendamment l'un de l'autre, un atome d'hydrogène, un groupe phényle, un groupe carbamoyle ou un groupe hydroxyméthyle ; Z représente un diradical alkylène non substitué, un diradical phényle ou un diradical hétéroarylène ; et x et y valent chacun, indépendamment l'un de l'autre, 0 ou 1.

**4.** 4,5-diamino-pyrazole selon la revendication 3, **caractérisé en ce que** R1 à R3 sont des atomes d'hydrogène.

**5.** 4,5-diamino-pyrazole selon l'une quelconque des revendications 1 à 4, **caractérisé en ce qu'**il est un sel de l'acide sulfurique, chlorhydrique, citrique ou tartrique.

**6.** Composition pour la teinture par oxydation de fibres kératiniques, **caractérisée en ce qu'**elle contient au moins un 4,5-diamino-pyrazole selon l'une quelconque des revendications 1 à 5.

**7.** Composition selon la revendication 6, **caractérisée en ce qu'**elle contient le 4,5-diamino-pyrazole en une quantité de 0,005 à 20 % en poids.

**8.** Composition selon la revendication 6 ou 7, **caractérisée en ce qu'**elle contient en outre d'autres développeurs et/ou coupleurs en une quantité totale, respectivement, de 0,01 à 20 % en poids.

**9.** Composition selon la revendication 6 ou 8, **caractérisée en ce qu'**elle contient en outre de 0,01 à 10 % en poids de colorants directs.

**10.** Composition selon l'une quelconque des revendications 6 à 9, **caractérisée en ce que** la matière colorante est mélangée avec l'oxydant en un rapport pondéral de 5:1 à 1:3.

**11.** Composition selon la revendication 10, **caractérisée en ce que** la composition de teinture par oxydation prête à l'emploi présente un pH de 3 à 11.